(19) Europäisches Patentamt
European Patent Office
Office européen des brevets

(11) Numéro de publication : **0 488 861 A1**

# (12) DEMANDE DE BREVET EUROPEEN

(21) Numéro de dépôt : **91403155.4**

(22) Date de dépôt : **22.11.91**

(51) Int. Cl.$^5$ : **C07B 41/04,** C07C 43/23, C07C 43/205, C07C 43/225

(30) Priorité : **29.11.90 FR 9014935**

(43) Date de publication de la demande : **03.06.92 Bulletin 92/23**

(84) Etats contractants désignés : **BE CH DE FR GB IT LI NL**

(71) Demandeur : **RHONE-POULENC CHIMIE**
**25, quai Paul Doumer**
**F-92408 Courbevoie Cédex (FR)**

(72) Inventeur : **Nobel, Dominique**
**132, allée du Chateau**
**F-69270 Fontaines-Saint-Martin (FR)**

(74) Mandataire : **Dutruc-Rosset, Marie-Claude et al**
**RHONE-POULENC CHIMIE Direction des Brevets 25, Quai Paul Doumer**
**F-92408 Courbevoie Cédex (FR)**

## (54) Procédé de préparation de composés aromatiques alkoxylés.

(57) La présente invention a pour objet un procédé de préparation de composés aromatiques alkoxylés. L'invention vise un procédé d'alkoxylation ou d'aralkoxylation de composés aromatiques halogénés.

Plus précisément, l'invention consiste en un procédé de préparation de composés aromatiques alkoxylés par réaction d'un composé aromatique mono- ou polyhalogéné avec un alcoolate de métal alcalin ou alcalino-terreux, en présence de cuivre et/ou d'un composé du cuivre caractérisé par le fait que l'on opère en présence d'une quantité efficace de dioxyde de carbone ou d'un composé susceptible de former du dioxyde de carbone dans le milieu réactionnel.

EP 0 488 861 A1

La présente invention a pour objet un procédé de préparation de composés aromatiques alkoxylés. Plus précisément, l'invention vise un procédé d'alkoxylation ou d'aralkoxylation de composés aromatiques halogénés.

Dans l'exposé qui suit de la présente invention, on désigne par "composé aromatique halogéné", tout composé aromatique portant au moins un atome d'halogène.

Par composé aromatique, on entend la notion classique d'aromaticité telle que définie dans la littérature, notamment par Jerry MARCH - Advanced Organic Chemistry, 3ème édition, John Wiley and Sons, 1985 p. 37 et suivantes.

Il est connu selon le brevet GB 2 089 672 d'introduire un ou plusieurs substituants alkoxy ou aralkoxy sur un noyau aromatique, en faisant réagir un composé mono- ou polyhalogéné avec un alcoolate de métal alcalin ou alcalino-terreux, en présence d'un catalyseur constitué d'un ester de l'acide formique dénommé "formiate" et d'un sel cuivreux. Dans ce procédé, le formiate utilisé est essentiellement le formiate de méthyle. Une difficulté majeure pour la mise en oeuvre d'un tel procédé réside dans l'utilisation du formiate de méthyle. En effet, ledit réactif n'est pas d'une manipulation aisée car c'est un produit très volatil à température ambiante et pose donc des problèmes de sécurité. Par ailleurs, il est à noter que dans le cas de la méthoxylation, par exemple, du bromobenzène par le méthylate de sodium, en présence de formiate de méthyle et de bromure cuivreux, la réaction est longue et incomplète car 44 % de bromobenzène n'ont pas réagi.

Un des objectifs de la présente invention est de fournir un procédé d'alkoxylation ou d'aralkoxylation de composés aromatiques mono- ou polyhalogénés qui permet d'éviter les inconvénients précités.

Plus précisément, l'invention consiste en un procédé de préparation de composés aromatiques alkoxylés ou aralkoxylés de formule générale (I) :

$$R_o - (O - R)_n \quad (I)$$

dans laquelle :

– n est un nombre entier compris entre 1 et 6,

– $R_o$ représente un radical cyclique aromatique ayant au moins 5 atomes dans le cycle, éventuellement substitué, et représentant au moins l'un des radicaux suivants :

. un radical carbocyclique aromatique, monocyclique ou polycyclique,

. un radical hétérocyclique aromatique, monocyclique ou polycyclique comportant au moins un des hétéroatomes O, N et S,

– R représente un radical hydrocarboné ayant de 1 à 12 atomes de carbone, qui peut être un radical aliphatique acyclique, saturé ou insaturé, linéaire ou ramifié ; un radical cycloaliphatique, saturé ou insaturé, monocyclique ou polycyclique ; un radical cycloaliphatique- ou arylaliphatique, saturé ou insaturé, linéaire ou ramifié,

procédé qui consiste à faire réagir :

– un composé aromatique mono- ou polyhalogéné répondant à la formule générale (II) :

$$R_o - (X)_n \quad (II)$$

dans laquelle :

– n est un nombre entier compris entre 1 et 6,

– X représente un atome d'iode, de brome ou de chlore,

– $R_o$ représente un radical cyclique aromatique ayant au moins 5 atomes dans le cycle, éventuellement substitué, et représentant au moins l'un des radicaux suivants :

. un radical carbocyclique aromatique, monocyclique ou polycyclique,

. un radical hétérocyclique aromatique, monocyclique ou polycyclique comportant au moins un des hétéroatomes O, N et S,

– et un alcoolate de métal alcalin ou alcalino-terreux de formule générale (III) :

$$M^{m+} \ [O - R]_m^- \quad (III)$$

dans laquelle :

– M représente un métal alcalin ou alcalino-terreux,

– m représente la valence du métal alcalin ou alcalino-terreux,

– R représente un radical hydrocarboné ayant de 1 à 12 atomes de carbone, qui peut être un radical aliphatique acyclique, saturé ou insaturé, linéaire ou ramifié ; un radical cycloaliphatique, saturé ou insaturé, monocyclique ou polycyclique ; un radical cycloaliphatique- ou arylaliphatique, saturé ou insaturé, linéaire ou ramifié,

en présence de cuivre et/ou d'un composé du cuivre caractérisé par le fait que l'on opère en présence d'une quantité efficace de dioxyde de carbone ou d'un composé susceptible de former du dioxyde de carbone dans le milieu réactionnel.

Conformément à la présente invention, il a été trouvé que la réaction d'alkoxylation ou d'aralkoxylation d'un composé mono- ou polyhalogéné était particulièrement activée par la présence d'un co-catalyseur qui, selon

une interprétation sans caractère limitatif, facilite ainsi la dissolution du cuivre en raison de la formation d'un complexe entre le dioxyde de carbone et le cuivre.

L'invention s'applique plus particulièrement aux composés aromatiques halogénés de formule (II) dans laquelle le radical $R_o$ symbolise :

1° - un radical carbocyclique aromatique, monocyclique ou polycyclique.

Par "radical carbocyclique polycyclique", on entend :

. un radical constitué par au moins 2 carbocycles aromatiques et formant entre eux des systèmes ortho ou ortho et péricondensés,

. un radical constitué par au moins 2 carbocycles dont l'un seul d'entre eux est aromatique et formant entre eux des systèmes ortho ou ortho et péricondensés.

2° - un radical hétérocyclique aromatique, monocyclique ou polycyclique.

Par "radical hétérocyclique polycyclique", on définit :

. un radical constitué par au moins 2 hétérocycles contenant au moins un hétéroatome dans chaque cycle dont au moins l'un des deux cycles est aromatique et formant entre eux des systèmes ortho- ou ortho- et péricondensés,

. un radical constitué par au moins un cycle hydrocarboné et au moins un hétérocycle dont au moins l'un des cycles est aromatique et formant entre eux des systèmes ortho- ou ortho- et péricondensés.

3° - un radical divalent constitué par un enchaînement de groupements, tels que définis aux paragraphes 1 et/ou 2 liés entre eux :

. par un lien valentiel,

. par un radical alkylène ou alkylidène ayant de 1 à 4 atomes de carbone, de préférence un radical méthylène ou isopropylidène,

. par l'un des groupes suivants :

$-O-$ ,  $-CO-$ ,

$-S-$ ,  $-SO-$ ,  $-SO_2-$ ,

$-N-$ ,  $-CO-N-$
|  |
$R_1$  $R_1$

dans ces formules, $R_1$ représentant un atome d'hydrogène ou un radical alkyle ayant de 1 à 4 atomes de carbone, un radical cyclohexyle ou phényle.

Comme exemples de radicaux listés sous 1° à 3°, on peut citer :

1° - les radicaux phényle, tolyle, xylyle ; les radicaux naphtyle, anthryle,

2° - les radicaux furyle, pyrrolyle, thiényle, isoxazolyle, furazannyle, isothiazolyle, imidazolyle, pyrazolyle, pyridyle, pyridazinyle, pyrimidinyle ; les radicaux quinolyle, naphtyridinyle, benzofurannyle, indolyle.

3° - les radicaux biphényle, méthylène-1,1′ biphényle, isopropylidène1,1′ biphényle, oxy-1,1′ biphényle, imino-1,1′ biphényle.

Comme mentionné précédemment, le radical $R_o$ qui est un composé aromatique porteur d'au moins un atome d'halogène, peut être également porteur d'un ou plusieurs autres substituants qui peuvent être un autre atome d'halogène ou de toute autre nature dans la mesure où ils ne gênent pas la réaction. Généralement, par plusieurs substituants, on définit moins de quatre substituants sur un noyau aromatique.

Comme exemples de substituants donnés à titre illustratif et sans caractère limitatif, on peut mentionner l'un des groupes ou fonctions suivantes :

. un radical de formule $-R_2-OH$ dans laquelle le radical $R_2$ représente un lien valentiel ou un radical hydrocarboné divalent, linéaire ou ramifié, saturé ou insaturé ayant de 1 à 4 atomes de carbone, tel que par exemple, méthylène, éthylène, propylène, isopropylène, isopropylidène,

. un radical alkyle, linéaire ou ramifié, ayant de 1 à 6 atomes de carbone, de préférence de 1 à 4 atomes de carbone, tel que méthyle, éthyle, propyle, isopropyle, butyle, isobutyle, sec-butyle, tert-butyle,

. un radical alcényle linéaire ou ramifié ayant de 2 à 6 atomes de carbone, de préférence, de 2 à 4 atomes de carbone, tel que vinyle, allyle,

. un radical alkoxy linéaire ou ramifié ayant de 1 à 6 atomes de carbone, de préférence de 1 à 4 atomes de carbone tel que les radicaux méthoxy, éthoxy, propoxy, isopropoxy, butoxy,

. un groupe acyle ayant de 2 à 6 atomes de carbone,

. un radical de formule $-R_2-COOH$, $R_2$ ayant la signification donnée précédemment,

. un radical de formule $-R_2-COOR_3$ dans laquelle $R_2$ a la signification donnée précédemment et $R_3$ représente un radical alkyle, linéaire ou ramifié, ayant de 1 à 6 atomes de carbone,

. un radical de formule $-R_2-NH_2$ avec un groupe $NH_2$ N-protégé, $R_2$ ayant la signification donnée précédemment,

. un radical de formule $-R_2-N(R'_3)$ dans laquelle $R_2$ a la signification donnée précédemment et les radicaux $R'_3$ identiques ou différents représentent un atome d'hydrogène ou un radical alkyle, linéaire ou ramifié, ayant de 1 à 6 atomes de carbone,

. un radical de formule $-R_2-CO-N(R'_3)$, $R_2$ et $R'_3$ ayant la signification donnée précédemment,

. un radical de formule $-R_2-Z$ dans laquelle $R_2$ a la signification donnée précédemment et Z symbolise un atome d'halogène X, tel que précédemment défini, un atome de fluor ou un groupe $CF_3$.

S'il y a présence de substituants sur le cycle aromatique, il y a lieu de veiller que celui-ci n'interfère pas au niveau du produit désiré.

De même, lors de la présence d'une fonction amine primaire, il peut être nécessaire de la N-protéger par un groupe protecteur, par exemple acyle et plus-spécialement acétyle.

Si le cycle est porteur d'un groupe du type $-R_2-COOR_3$, le groupe $R_3$ sera échangé par le groupe R provenant de l'alcoolate de métal alcalin ou alcalino-terreux, si $R_3$ et R sont des groupes alkyles de nature différente.

Si le cycle est porteur d'une fonction acide du type $-R_2-COOH$, le groupe sera salifié et entraînera une consommation supplémentaire de l'alcoolate utilisé.

Lorsque le cycle aromatique est porteur d'une chaîne aliphatique latérale avec présence d'un atome d'halogène X, celui-ci peut être substitué également par le groupe OR provenant de l'alcoolate de métal alcalin ou alcalino-terreux et il y aura donc lieu d'en tenir compte pour la détermination des quantités de réactifs à mettre en oeuvre.

Il est à noter que la présente invention n'inclut pas dans la définition des composés aromatiques halogénés de formule (II), les composés aromatiques halogénés porteurs d'une fonction aldéhyde. Ceux-ci font l'objet des demandes de brevets FR 89/133 70 et EP 90 402786.9.

Parmi les composés répondant à la formule générale (II), l'invention s'applique plus particulièrement aux composés aromatiques halogénés répondant aux formules (IIa) et (IIb) :

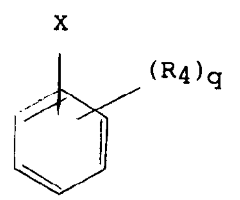

(IIa)

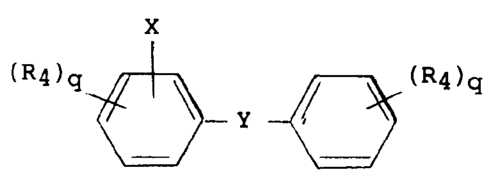

(IIb)

dans lesdites formules (IIa) et (IIb) :

– q identiques ou différents représentent un nombre entier compris entre 0 et 4, de préférence compris entre 0 et 3,

– X représente un atome d'halogène, de préférence un atome d'iode, de brome ou de chlore, de préférence un atome de brome

– $R_4$ identiques ou différents représentent :

. un atome d'hydrogène,

. un atome d'iode, de brome ou de chlore, de préférence un atome de brome,

. un radical alkyle linéaire ou ramifié ayant de 1 à 4 atomes de carbone, de préférence un radical méthyle ou éthyle,

. un radical alkoxy linéaire ou ramifié ayant de 1 à 4 atomes de carbone, de préférence, un radical méthoxy ou éthoxy,

. un radical hydroxyle,

. un radical -COO $R_5$ dans lequel le radical $R_5$ représente un radical alkyle linéaire ou ramifié ayant de 1 à 4 atomes de carbone,

. un groupe $-NH_2$ de préférence N-protégé,

. un groupe $-CF_3$,

– Y symbolise un lien valentiel, un radical alkylène ou alkylidène ayant de 1 à 4 atomes de carbone, un groupe -O-.

A titre d'exemples de composés aromatiques halogénés répondant à la formule générale (II), on peut citer plus particulièrement :

– le bromobenzène
– les dibromobenzènes
– les tribromozènes
– le tétrabromo-1,2,4,5 benzène
– l'hexabromobenzène
– le bromo-2 mésitylène
– le bromo-5 triméthyl-1,2,4 benzène
– le bromo-1-tert-butyl-4 benzène
– les bromostyrènes
– le bromo-2 chlorobenzène
– le bromo-3 chlorobenzène
– le bromo-4 chlorobenzène
– le bromo-2 chloro-5 toluène
– les bromodichlorobenzènes
– le bromo-5 fluoro-2 toluène
– les bromo (trifluorométhyl)benzènes
– les bromofluorobenzènes
– les bromodifluorobenzènes
– les bromotrifluorobenzènes
– les bromotétrafluorobenzènes
– le bromo-1 nitro-2 benzène
– le bromo-1 nitro-4 benzène
– le bromo-2 nitro-4 toluène
– le bromo-2 nitro-5 toluène
– le bromo-2 nitro-6 toluène
– le bromo-1 naphtalène
– le bromo-2 naphtalène
– le bromo-9 anthracène
– le bromo-1 méthyl-2 naphtalène
– le bromo-1 méthyl-4 naphtalène
– le bromo-2 méthoxy-6 naphtalène
– le bromo-2 phénol
– le bromo-3 phénol
– le bromo-4 phénol
– le bromo-1 naphtol-2
– le bromo-6 naphtol-2
– le bromo-2 méthyl-4 phénol
– le bromo-4 diméthyl-2,6 phénol
– le bromo-4 diméthyl-3,5 phénol
– le dibromo-2,6 méthyl-4 phénol
– le bromo-2-p-crésol
– le bromo-2 chloro-4 phénol
– le bromo-4 chloro-2 phénol
– le bromo-4 chloro-6-o-crésol
– les bromofluorophénols
– le bromo-2 anisole
– le bromo-3 anisole

– le bromo-4 anisole
– le dibromo-2,6 méthyl-4 anisole
– l'$\alpha,\alpha,\alpha$ trifluorobromoanisole
– les bromofluoroanisoles
– le bromo-4 phénétole
– le bromo-1 diméthoxy-2,4 benzène
– le bromo-1 diméthoxy-2,5 benzène
– l'acide bromo-2 benzoïque
– l'acide bromo-3 benzoïque
– l'acide bromo-4 benzoïque
– l'acide bromo-4 dihydroxy-3,5 benzoïque
– l'acide bromo-5 dihydroxy-2,4 benzoïque
– l'acide bromo-3 méthyl-4 benzoïque
– l'ester méthylique de l'acide (dibromo-3,5 acétamido-4) benzoïque
– l'ester éthylique de l'acide (dibromo-3,5 acétamido-4) benzoïque
– l'ester méthylique de l'acide (dibromo-3,5 méthoxy-4) benzoïque
– l'ester éthylique de l'acide (dibromo-3,5 méthoxy-4) benzoïque
– l'ester méthylique de l'acide (dibromo-3,5 éthoxy-4) benzoïque
– l'ester éthylique de l'acide (dibromo-3,5 éthoxy-4) benzoïque
– la bromo-2′ acétophénone
– la bromo-3′ acétophénone
– la bromo-4′ acétophénone
– la bromo-2 acétanilide
– la bromo-3 acétanilide
– la bromo-4 acétanilide
– les bromométhylacétanilides
– les bromofluoroacétanilides
– la bromo-4 (trifluorométhyl)-2 acétanilide
– la bromo-N,N-diméthylacétanilide
– la bromo-4 chloro-2 acétanilide
– les bromodifluoroacétanilides
– le bromo-2 benzonitrile
– le bromo-3 benzonitrile
– le bromo-4 benzonitrile
– le bromo-2 benzamide
– le bromo-4 benzamide
– les bromothiophénols
– le bromo-4 diphényléther
– le bromo-4 benzophénone
– les biphénols mono- ou polybromés
– le bromo-4 nitro-3 biphényl
– les bromo bis-phénols
– les bromo isopropylidène-4,4′ bis-phénols
– le bromo-4 méthyl-3 pyrazole
– le bromo-4 diméthyl-3,5 pyrazole
– le bromo-3 furane
– le bromo-5 indole
– le bromo-2 thiophène
– le bromo-3 thiophène
– la bromo-2 pyridine
– la bromo-3 pyridine
– la bromo-4 pyridine
– le bromo-4 isoquinoléïne
– le bromo-4 (méthylènedioxy)-1,2 benzène

Parmi les composés répondant à la formule générale (II), l'invention s'applique tout particulièrement aux composés aromatiques halogénés suivants :

– le bromobenzène
– les dibromobenzènes

– les tribromozènes
– le tétrabromo-1,2,4,5 benzène
– l'hexabromobenzène
– les bromostyrènes
– le bromo-2 chlorobenzène
– les bromofluorobenzènes
– les bromodifluorobenzènes
– les bromotrifluorobenzènes
– les bromotétrafluorobenzènes
– le bromo-2 phénol
– le bromo-3 phénol
– le bromo-4 phénol
– le dibromo-2,6 méthyl-4 phénol
– le bromo-2 anisole
– le bromo-3 anisole
– le bromo-4 anisole
– le dibromo-2,6 méthyl-4 anisole
– l'acide bromo-2 benzoïque
– l'acide bromo-3 benzoïque
– l'acide bromo-4 benzoïque
– l'ester méthylique de l'acide (dibromo-3,5 acétamido-4) benzoïque
– l'ester éthylique de l'acide (dibromo-3,5 acétamido-4) benzoïque
– l'ester méthylique de l'acide (dibromo-3,5 méthoxy-4) benzoïque
– l'ester éthylique de l'acide (dibromo-3,5 méthoxy-4) benzoïque
– l'ester méthylique de l'acide (dibromo-3,5 éthoxy-4) benzoïque
– l'ester éthylique de l'acide (dibromo-3,5 éthoxy-4) benzoïque
– la bromo-4 (trifluorométhyl)-2 acétanilide
– les biphényls mono- ou polybromés
– les bromo bis-phénols
– les bromo isopropylidène-4,4′ bis-phénols
– le bromo-2 thiophène
– le bromo-3 thiophène
– la bromo-2 pyridine
– la bromo-3 pyridine
– la bromo-4 pyridine

L'alcoolate de métal alcalin ou alcalino-terreux qui intervient dans le procédé de l'invention répond à la formule (III) dans laquelle R représente :

1° - un radical alkyle, alcényle, alcadiényle, alcynyle linéaire ou ramifié ayant, de préférence, moins de 6 atomes de carbone,

2° - un radical cycloalkyle ou cycloalcényle ayant, de préférence, de 5 à 7 atomes de carbone et l'on peut citer en particulier le radical cyclohexyle,

3° - un radical aliphatique acyclique, saturé ou insaturé, linéaire ou ramifié tel que précisé sous 1°, porteur d'un substituant cyclique. Par cycle, on entend un cycle carbocyclique saturé, insaturé ou aromatique. Comme exemple, on peut mentionner le radical benzyle.

Le terme "alcoolate" est utilisé dans le présent texte de manière générique et désigne également les aralkoxydes métalliques.

Parmi les alcoolates précités, on met en oeuvre de préférence dans le procédé de l'invention, les alcoolates de métaux alcalins et plus particulièrement les alcoolates de sodium ou de potassium des alcanols primaires ou secondaires ayant 1 à 4 atomes de carbone conviennent particulièrement bien.

Les plus fréquemment utilisés sont le méthylate de sodium et l'éthylate de sodium.

Conformément au procédé de l'invention, on effectue la réaction d'alkoxylation ou d'aralkoxylation du composé halogéné de formule (II) en le faisant réagir avec un alcoolate de métal alcalin ou alcalinoterreux, en présence d'un catalyseur au cuivre et d'un co-catalyseur qui est le dioxyde de carbone ou un composé susceptible de le former dans le milieu réactionnel.

Le dioxyde de carbone peut être mis en oeuvre en solution dans le milieu liquide réactionnel par circulation dans celui-ci.

Comme exemples de composés susceptibles de former du dioxyde de carbone dans le milieu réactionnel, on peut citer :

– les α(ou β)cétoesters et les α(ou β)cétoacides ;
– les carbonates d'amine, les urées et les carbodiimides ;
– les acides dicarboxyliques comme l'acide malonique, les acides α-cyano- ou α-nitro-carboxyliques ;
– les acides carboxyliques βγ-insaturés.

Les composés du cuivre servant de catalyseurs sont connus. Ce sont d'une manière générale tous les composés organiques ou inorganiques du cuivre I ou du cuivre II.

Le cuivre métal peut être utilisé, mais son action est plus lente car il faut préalablement qu'il se transforme en partie en cuivre I ou cuivre II.

A titre non limitatif, on peut citer comme composé du cuivre le chlorure cuivreux, le chlorure cuivrique, le carbonate de cuivre II basique, le nitrate cuivreux, le nitrate cuivrique, le sulfate cuivrique, l'acétate cuivrique, le trifluorométhylsulfonate cuivrique, l'hydroxyde cuivrique, le picolinate de cuivre II, le méthylate de cuivre 1, le méthylate de cuivre II, le chélate de cuivre II de la 8-quinoléine, les composés de cuivre de formule $ClCuOCH_3$, $Cu_2(OCH_3)_2(acac)_2$.

Conformément au procédé de l'invention, l'alkoxylation ou l'aralkoxylation du composé aromatique halogéné répondant à la formule (II) est conduite en milieu organique constitué, le plus souvent, par l'alcanol correspondant à l'alcoolate de métal alcalin ou alcalino-terreux utilisé.

On choisit, préférentiellement comme solvant, le méthanol ou l'éthanol.

Lorsque l'alcoolate de métal alcalin ou alcalino-terreux présente une condensation en carbone supérieure à 4 atomes de carbone, il est souhaitable de faire appel à un solvant inerte vis-à-vis de la réaction et de choisir, de préférence, un solvant aprotique polaire.

Comme exemples de solvants aprotiques polaires convenant à la mise en oeuvre du procédé de l'invention, on peut mentionner, plus particulièrement les éthers et, plus précisément, les éthers diméthyliques dérivant de l'oxyde d'éthylène ou de l'oxyde de propylène tels que le diméthyléther de l'éthylèneglycol (ou diméthoxy-1,2 éthane), le diméthyléther du diéthylèneglycol (ou diméthoxy-1,5 oxa-3 pentane), le diméthoxy-1,8 dioxa-3,6 octane, le diméthoxy-1,11 trioxa-3,6,9 undécane, le diméthoxy-1,2 méthyl-1 éthane, le diméthoxy-1,5 diméthyl-1,4 oxa-3 pentane, le diméthoxy-1,7 diméthyl-1,4 dioxa-3,6 octane.

On peut également utiliser plusieurs solvants

Parmi tous les solvants précités, le diméthyléther de l'éthylène-glycol et le diméthyléther du diéthylèneglycol sont préférés.

La concentration du composé de formule (II) exprimée en poids dudit composé (II) par rapport au poids total composé (II) + solvant est généralement de 3 à 40 % et, de préférence, de 10 à 30 %.

La quantité d'alcoolate de métal alcalin ou alcalino-terreux utilisée est égale ou supérieure à la quantité stoechiométrique nécessaire, d'une part pour transformer le ou les atomes d'halogène en groupements alkoxy (ou aralkoxy) et d'autre part pour transformer le composé de formule (II) en phénate de métal alcalin ou alcalino-terreux dans le cas où ledit composé présente un ou plusieurs radicaux hydroxyle. On effectue donc la salification de ou des groupements OH.

Généralement, l'alcoolate de métal alcalin ou alcalino-terreux est mis en oeuvre en une quantité correspondant à la quantité stoechiométrique nécessaire pour salifier le ou les groupes hydroxyle plus la quantité égale de 1 à 5 fois, et de préférence de 1 à 3 fois, la quantité stoechiométrique nécessaire pour transformer le ou les atomes d'halogène en groupements alkoxy (ou aralkoxy).

La concentration de l'alcoolate de métal alcalin ou alcalinoterreux est avantageusement supérieure à 1 mole/litre et, de préférence, comprise entre 1 et 5 mole/litre.

Il est à noter que la borne supérieure ne présente aucun caractère critique.

De manière pratique, l'alcoolate de métal alcalin ou alcalinoterreux est formé in situ, en faisant réagir un excès d'alcanol avec le métal alcalin ou alcalino-terreux choisi.

La quantité de catalyseur au cuivre utilisée dans le procédé de l'invention peut varier très largement.

Habituellement, le rapport molaire catalyseur au cuivre/composé de formule (II) est de 1 à 50 % et, de préférence, de 2 % à 20 %.

La quantité de co-catalyseur est telle que l'on ait un rapport molaire co-catalyseur / catalyseur au cuivre de 1 à 10 et, de préférence, de 1 à 5.

La température de la réaction d'alkoxylation est généralement comprise entre 60°C et 220°C, et, de préférence de 100°C à 180°C.

De préférence, on choisit la température de reflux du mélange réactionnel

La pression n'est pas un paramètre critique en soi, mais pour atteindre les températures indiquées précédemment et ne pas perdre de solvant, le procédé est habituellement réalisé sous pression autogène.

La durée de la réaction d'alkoxylation ou d'aralkoxylation peut varier largement selon la nature du substrat. Elle dure généralement entre 1 et 10 heures, de préférence entre 2 et 5 heures.

Le procédé de l'invention est parfaitement bien adapté à la préparation des composés aromatiques mono-

ou polyalkoxylés suivants :

- le méthoxybenzène
- les diméthoxybenzènes
- les triméthoxybenzènes
- le tétraméthoxy-1,2,4,5 benzène
- l'hexaméthoxybenzène
- les méthoxystyrènes
- le méthoxy-2 chlorobenzène
- les méthoxyfluorobenzènes
- les méthoxydifluorobenzènes
- les méthoxytrifluorobenzènes
- les méthoxytétrafluorobenzènes
- le méthoxy-2 phénol
- le méthoxy-3 phénol
- le méthoxy-4 phénol
- le diméthoxy-2,6 méthyl-4 phénol
- le méthoxy-2 anisole
- le méthoxy-3 anisole
- le méthoxy-4 anisole
- le diméthoxy-2,6 méthyl-4 anisole
- l'acide méthoxy-2 benzoïque
- l'acide méthoxy-3 benzoïque
- l'acide méthoxy-4 benzoïque
- l'ester méthylique de l'acide (diméthoxy-3,5 acétamido-4) benzoïque
- l'ester éthylique de l'acide (diéthoxy-3,5 acétamido-4) benzoïque
- l'ester méthylique de l'acide (triméthoxy-3,4,5) benzoïque
- l'ester éthylique de l'acide (diéthoxy-3,5 méthoxy-4) benzoïque
- l'ester méthylique de l'acide (diméthoxy-3,5 éthoxy-4) benzoïque
- l'ester éthylique de l'acide (triéthoxy-3,4,5) benzoïque
- la méthoxy-4 (trifluorométhyl)-2 acétanilide
- les biphényls mono- ou polyméthoxylés
- les biphényls mono- ou polyéthoxylés
- les bis-phénols méthoxylés ou éthoxylés
- les méthoxyisopropylidène-4,4′ bis-phénols
- le méthoxy-2 thiophène
- le méthoxy-3 thiophène
- la méthoxy-2 pyridine
- la méthoxy-3 pyridine
- la méthoxy-4 pyridine

Un des avantages particulièrement intéressant du procédé de l'invention est qu'il permet d'effectuer la réaction d'alkoxylation ou d'aralkoxylation avec un excellent taux de transformation du composé halogéné de départ.

Un autre avantage du procédé de l'invention est qu'il fait appel, dans sa forme préférentielle, à un solvant courant, peu onéreux et facilement recyclable.

Les exemples qui suivent illustrent l'invention.

Dans les exemples, la signification des abréviations est la suivante :

$$RT = \frac{\text{nombre de moles de composé aromatiques alkoxylé formées}}{\text{nombre de mole de composé aromatique halogéné transformées}} \%$$

EXEMPLE 1

Méthoxylation du bromo-2 phénol en méthoxy-2 phénol (gaïacol)

Dans un réacteur téfloné de 40 $cm^3$, muni d'un système de chauffage et d'une agitation, on charge :

- 1,73 g (10 mmoles) de bromo-2 phénol
- 2,2 g (40 mmoles) de méthylate de sodium
- 0,110 g (0,5 mmole) de carbonate de cuivre basique $Cu(OH)_2CuCO_3$
- 21 $cm^3$ de méthanol

On fait buller dans cette suspension, du dioxyde de carbone anhydre pendant trente secondes, puis on chauffe 3 heures à 125°C sous agitation et sous pression autogène. On refroidit à température ambiante. On dilue à l'eau distillée, on ajuste le pH du mélange réactionnel à 4 à l'aide d'acide sulfurique concentré. On filtre la partie insoluble.

On dose par chromatographie en phase liquide, 1,05 g de gaïacol, ce qui correspond à un rendement (RT) de 85 %.

Essai comparatif A

On répète l'exemple 1, avec les mêmes charges, les mêmes conditions opératoires, mais sans dioxyde de carbone. On obtient un rendement de 39 % en gaïacol.

EXEMPLE 2

Méthoxylation du p-bromophénol en p-méthoxyphénol

On répète l'exemple 1, avec les mêmes charges et les mêmes conditions opératoires, mais en remplaçant le bromo-2 phénol par le p-bromophénol.

Après traitement, on obtient un rendement (RT) de 70 % en p-méthoxyphénol.

Essai comparatif B

On reproduit l'exemple 2, avec les mêmes charges et les mêmes conditions opératoires, mais sans dioxyde de carbone.

Après traitement, on obtient un rendement (RT) de 26 % en p-méthoxyphénol.

EXEMPLE 3

Méthoxylation du bromo-2 anisole en méthoxy-2 anisole (vératrole)

On répète l'exemple 1, avec les mêmes charges et les mêmes conditions opératoires, mais en remplaçant le bromo-2 phénol par 1,88 g (10 mmoles) de bromo-2 anisole.

On chauffe 3 heures, à 120°C

Après traitement, on dose par chromatographie en phase gazeuse, 1,22 g de vératrole, ce qui correspond à un rendement (RT) de 87 %.

EXEMPLE 4

Méthoxylation du bromo-4 anisole en diméthoxy-1,4 benzène

On répète l'exemple 3, avec les mêmes charges, les mêmes conditions opératoires, mais en remplaçant le bromo-2 anisole par le bromo-4 anisole.

Après traitement, on dose 1,30 g de diméthoxy-1,4 benzène, ce qui correspond à un rendement de 93 %.

EXEMPLE 5

Méthoxylation du tribromo-1,3,5 benzène en triméthoxy-1,3,5 benzène

On répète l'exemple 1, avec les mêmes charges et les mêmes conditions opératoires, mais en remplaçant le bromo-2 phénol par 3,14 g (10 mmoles) de tribromo-1,3,5 benzène et en utilisant 3,24 g (60 mmoles) de méthylate de sodium.

Après 12 heures de réaction à 125°C et après traitement, on obtient 1,09 g de triméthoxy-1,3,5 benzène, ce qui correspond à un rendement (RT) de 65 %.

EXEMPLE 6

Méthoxylation du bromo-4 fluorobenzène en fluoro-4 anisole

Dans un réacteur téfloné de 40 $cm^3$ muni d'un système de chauffage et d'une agitation, on charge :
– 1,75 g (10 mmoles) de bromo-4 fluorobenzène
– 5,40 g (30 mmoles) d'une solution de méthylate de sodium à 30 % dans le méthanol
– 0,110 g (0,5 mmole) de carbonate de cuivre basique
– 7 $cm^3$ de méthanol
Puis, on procède comme dans l'exemple 1.
On chauffe 6 heures à 115°C.
Après traitement, on dose par chromatographie en phase gazeuse, 1,16 g de fluoro-4 anisole, ce qui correspond à un rendement (RT) de 91,6 %.

EXEMPLE 7

Méthoxylation du bromobenzène en anisole

Dans un réacteur téfloné de 40 $cm^3$ muni d'un système de chauffage et d'une agitation, on charge :
– 1,57 g (10 mmoles) de bromobenzène
– 2,71 g (15 mmoles) d'une solution de méthylate de sodium à 30 % dans le méthanol
– 0,110 g (0,5 mmole) de carbonate de cuivre basique
Puis, on procède comme dans l'exemple 1.
On chauffe 5 heures 30 à 125°C.
Après traitement, on dose par chromatographie en phase gazeuse, 0,33 g d'anisole, ce qui correspond à un rendement (RT) de 91,5 %.

**Revendications**

**1 -** Procédé de préparation de composés aromatiques alkoxylés ou aralkoxylés de formule générale (I) :

$$R_o - (O - R)_n \quad (I)$$

dans laquelle :
– n est un nombre entier compris entre 1 et 6,
– $R_o$ représente un radical cyclique aromatique ayant au moins 5 atomes dans le cycle, éventuellement substitué, et représentant au moins l'un des radicaux suivants :
. un radical carbocyclique aromatique, monocyclique ou polycyclique,
. un radical hétérocyclique aromatique, monocyclique ou polycyclique comportant au moins un des hétéroatomes O, N et S,
– R représente un radical hydrocarboné ayant de 1 à 12 atomes de carbone, qui peut être un radical aliphatique acyclique, saturé ou insaturé, linéaire ou ramifié ; un radical cycloaliphatique, saturé ou insaturé, monocyclique ou polycyclique ; un radical cycloaliphatique- ou arylaliphatique, saturé ou insaturé, linéaire ou ramifié,

procédé qui consiste à faire réagir :
– un composé aromatique mono- ou polyhalogéné répondant à la formule générale (II) :

$$R_o -(X)_n \quad (II)$$

dans laquelle :
– n est un nombre entier compris entre 1 et 6,
– X représente un atome d'iode, de brome ou de chlore,
– $R_o$ représente un radical cyclique aromatique ayant au moins 5 atomes dans le cycle, éventuellement substitué, et représentant au moins l'un des radicaux suivants :
. un radical carbocyclique aromatique, monocyclique ou polycyclique,
. un radical hétérocyclique aromatique, monocyclique ou polycyclique comportant au moins un des hétéroatomes O, N et S,
– et un alcoolate de métal alcalin ou alcalino-terreux de formule générale (III) :

$$M^{m+} \; [O - R]_m^- \quad (III)$$

dans laquelle :
– M représente un métal alcalin ou alcalino-terreux,

– m représente la valence du métal alcalin ou alcalino-terreux,

– R représente un radical hydrocarboné ayant de 1 à 12 atomes de carbone, qui peut être un radical aliphatique acyclique, saturé ou insaturé, linéaire ou ramifié ; un radical cycloaliphatique, saturé ou insaturé, monocyclique ou polycyclique ; un radical cycloaliphatique- ou arylaliphatique, saturé ou insaturé, linénéaire ou ramifié,

en présence de cuivre et/ou d'un composé du cuivre caractérisé par le fait que l'on opère en présence d'une quantité efficace de dioxyde de carbone ou d'un composé susceptible de former du dioxyde de carbone dans le milieu réactionnel.

**2 -** Procédé selon la revendication 1 caractérisé par le fait que le composé aromatique halogéné répond à la formule générale (II) dans laquelle le radical $R_o$ symbolise :

1° - un radical carbocyclique aromatique, monocyclique ou polycyclique.

2° - un radical hétérocyclique aromatique, monocyclique ou polycyclique.

3° - un radical divalent constitué par un enchaînement de groupements, tels que définis aux paragraphes 1 et/ou 2 liés entre eux :

. par un lien valentiel,

. par un radical alkylène ou alkylidène ayant de 1 à 4 atomes de carbone, de préférence un radical méthylène ou isopropylidène,

. par l'un des groupes suivants :

$-O-$ , $-CO-$ ,

$-S-$ , $-SO-$ , $-SO_2-$ ,

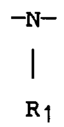 , 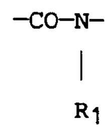

dans ces formules, $R_1$ représentant un atome d'hydrogène ou un radical alkyle ayant de 1 à 4 atomes de carbone, un radical cyclohexyle ou phényle.

**3 -** Procédé selon l'une des revendications 1 et 2 caractérisé par le fait que le composé aromatique halogéné répondant à la formule générale (II) est substitué par un ou plusieurs substituants tels que :

. un radical de formule $-R_2-OH$ dans laquelle le radical $R_2$ représente un lien valentiel ou un radical hydrocarboné divalent, linéaire ou ramifié, saturé ou insaturé ayant de 1 à 4 atomes de carbone, tel que par exemple, méthylène, éthylène, propylène, isopropylène, isopropylidène,

. un radical alkyle, linéaire ou ramifié, ayant de 1 à 6 atomes de carbone, de préférence de 1 à 4 atomes de carbone, tel que méthyle, éthyle, propyle, isopropyle, butyle, isobutyle, sec-butyle, tert-butyle,

. un radical alcényle linéaire ou ramifié ayant de 2 à 6 atomes de carbone, de préférence, de 2 à 4 atomes de carbone, tel que vinyle, allyle,

. un radical alkoxy linéaire ou ramifié ayant de 1 à 6 atomes de carbone, de préférence de 1 à 4 atomes de carbone tel que les radicaux méthoxy, éthoxy, propoxy, isopropoxy, butoxy,

. un groupe acyle ayant de 2 à 6 atomes de carbone,

. un radical de formule $-R_2-COOH$, $R_2$ ayant la signification donnée précédemment,

. un radical de formule $-R_2-C00R_3$ dans laquelle $R_2$ a la signification donnée précédemment et $R_3$ représente un radical alkyle, linéaire ou ramifié, ayant de 1 à 6 atomes de carbone,

. un radical de formule $-R_2-NH_2$ avec un groupe $NH_2$ N-protégé, $R_2$ ayant la signification donnée précédemment,

. un radical de formule $-R_2-N(R'_3)$ dans laquelle $R_2$ a la signification donnée précédemment et les radicaux $R'_3$ identiques ou différents représentent un atome d'hydrogène ou un radical alkyle, linéaire ou ramifié, ayant de 1 à 6 atomes de carbone,

. un radical de formule $-R_2-CO-N(R'_3)$, $R_2$ et $R'_3$ ayant la signification donnée précédemment,

. un radical de formule $-R_2-Z$ dans laquelle $R_2$ a la signification donnée précédemment et Z symbolise un atome d'halogène X, tel que précédemment défini, un atome de fluor ou un groupe $CF_3$.

**4 -** Procédé selon l'une des revendications 1 à 3 caractérisé par le fait que le composé aromatique halogéné répond à l'une des formules (IIa) ou (IIb) :

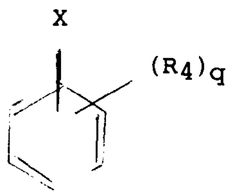

(IIa)

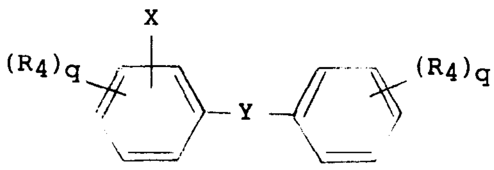

(IIb)

dans lesdites formules (IIa) et (IIb) :

– q identiques ou différents représentent un nombre entier compris entre 0 et 4, de préférence compris entre 0 et 3,

– X représente un atome d'halogène, de préférence un atome d'iode, de brome ou de chlore, de préférence un atome de brome

– $R_4$ identiques ou différents représentent :

. un atome d'hydrogène,

. un atome d'iode, de brome ou de chlore, de préférence un atome de brome,

. un radical alkyle linéaire ou ramifié ayant de 1 à 4 atomes de carbone, de préférence un radical méthyle ou éthyle,

. un radical alkoxy linéaire ou ramifié ayant de 1 à 4 atomes de carbone, de préférence, un radical méthoxy ou éthoxy,

. un radical hydroxyle,

. un radical -COO $R_5$ dans lequel le radical $R_5$ représente un radical alkyle linéaire ou ramifié ayant de 1 à 4 atomes de carbone,

. un groupe $-NH_2$ de préférence N-protégé,

. un groupe $-CF_3$,

– Y symbolise un lien valentiel, un radical alkylène ou alkylidène ayant de 1 à 4 atomes de carbone, un groupe -O-.

**5 -** Procédé selon d'une des revendications 1 à 4 caractérisé par le fait que le composé aromatique halogéné (II)) est choisi parmi :

– le bromobenzène
– les dibromobenzènes
– les tribromozènes
– le tétrabromo-1,2,4,5 benzène
– l'hexabromobenzène
– les bromostyrènes
– le bromo-2 chlorobenzène
– les bromofluorobenzènes
– les bromodifluorobenzènes
– les bromotrifluorobenzènes
– les bromotétrafluorobenzènes
– le bromo-2 phénol
– le bromo-3 phénol
– le bromo-4 phénol
– le dibromo-2,6 méthyl-4 phénol
– le bromo-2 anisole
– le bromo-3 anisole
– le bromo-4 anisole
– le dibromo-2,6 méthyl-4 anisole
– l'acide bromo-2 benzoïque
– l'acide bromo-3 benzoïque
– l'acide bromo-4 benzoïque
– l'ester méthylique de l'acide (dibromo-3,5 acétamido-4) benzoïque
– l'ester éthylique de l'acide (dibromo-3,5 acétamido-4) benzoïque
– l'ester méthylique de l'acide (dibromo-3,5 méthoxy-4) benzoïque
– l'ester éthylique de l'acide (dibromo-3,5 méthoxy-4) benzoïque
– l'ester méthylique de l'acide (dibromo-3,5 éthoxy-4) benzoïque

– l'ester éthylique de l'acide (dibromo-3,5 éthoxy-4) benzoïque
– la bromo-4 (trifluorométhyl)-2 aniline
– les biphényls mono- ou polybromés
– les bromo bis-phénols
– les bromo isopropylidène-4,4′ bis-phénols
– le bromo-2 thiophène
– le bromo-3 thiophène
– la bromo-2 pyridine
– la bromo-3 pyridine
– la bromo-4 pyridine

**6 -** Procédé selon l'une des revendications 1 à 5 caractérisé par le fait que l'alcoolate de métal alcalin ou alcalino-terreux répond à la formule générale (III) dans laquelle R représente un radical alkyle, alcényle, alcadiényle, alcynyle linéaire ou ramifié ayant de préférence moins de 6 atomes de carbone ou un radical cyclohexyle ou benzyle.

**7 -** Procédé selon l'une des revendications 1 à 6 caractérisé par le fait que l'alcoolate de métal alcalin est un alcoolate de sodium ou de potassium d'un alcanol primaire ou secondaire ayant 1 à 4 atomes de carbone.

**8 -** Procédé selon l'une des revendications 1 à 7 caractérisé par le fait que l'alcoolate de métal alcalin est le méthylate de sodium ou l'éthylate de sodium.

**9 -** Procédé selon l'une des revendications 1 à 8 caractérisé par le fait que l'on opère en présence d'un composé susceptible de former du dioxyde de carbone dans le milieu réactionnel choisi parmi :
– les $\alpha$(ou $\beta$)cétoesters et les $\alpha$(ou $\beta$)cétoacides ;
– les carbonates d'amine, les urées et les carbodiimides ;
– les acides dicarboxyliques comme l'acide malonique, les acides $\alpha$-cyano- ou $\alpha$-nitro-carboxyliques ;
– les acides carboxyliques $\beta\gamma$-insaturés.

**10 -** Procédé selon l'une des revendications 1 à 9 caractérisé par le fait que le catalyseur au cuivre est le cuivre métal ou un composé du cuivre qui est un composé organique ou inorganique du cuivre I ou du cuivre II, choisi parmi le chlorure cuivreux, le chlorure cuivrique, le carbonate de cuivre II basique, le nitrate cuivreux, le nitrate cuivrique, le sulfate cuivrique, l'acétate cuivrique, le trifluorométhylsulfonate cuivrique, l'hydroxyde cuivrique, le picolinate de cuivre II, le méthylate de cuivre I, le méthylate de cuivre II, le chélate de cuivre II de la 8-quinoléine, les composés du cuivre de formule $ClCuOCH_3$, $Cu_2(OCH_3)_2(acac)_2$.

**11 -** Procédé selon l'une des revendications 1 à 10 caractérisé par le fait que la réaction est conduite dans un solvant constitué par l'alcanol correspondant à l'alcoolate de métal alcalin ou alcalino-terreux utilisé.

**12 -** Procédé selon la revendication 11 caractérisé par le fait que la concentration initiale de composé de formule (II) par rapport au mélange composé de formule (II)/solvant est de 3 % à 40 % en poids par poids et de préférence de 10 % à 30 %.

**13 -** Procédé selon l'une des revendications 1 à 12 caractérisé par le fait que la quantité d'alcoolate de métal alcalin ou alcalino-terreux utilisée est égale ou supérieure à la quantité stoechiométrique nécessaire pour transformer le composé de formule (II) en phénate alcalin ou alcalino-terreux correspondant et pour transformer le ou les atomes d'halogène qu'il comporte en groupements alkoxy (ou aralkoxy).

**14 -** Procédé selon l'une des revendications 1 à 13 caractérisé par le fait que l'alcoolate de métal alcalin ou alcalino-terreux est mis en oeuvre en une quantité correspondant à la quantité stoechiométrique nécessaire pour salifier le ou les groupes hydroxyle plus la quantité égale de 1 à 5 fois, et de préférence de 1 à 3 fois, la quantité stoechiométrique nécessaire pour transformer le ou les atomes d'halogène en groupements alkoxy (ou aralkoxy).

**15 -** Procédé selon l'une des revendications 1 à 14 caractérisé par le fait que la concentration en alcoolate de métal alcalin ou alcalinoterreux est supérieure à 1 mole/litre, de préférence, comprise entre 1 et 5 moles/litre.

**16 -** Procédé selon l'une des revendications 1 à 15 caractérisé par le fait que le rapport molaire catalyseur au cuivre/composé de formule (II) est de 1 % à 50 % et de préférence de 2 % à 20 %.

**17 -** Procédé selon l'une des revendications 1 à 16 caractérisé par le fait que le rapport molaire co-catalyseur/catalyseur au cuivre est de 1 à 10 et de préférence de 1 à 5.

**18 -** Procédé selon l'une des revendications 1 à 17 caractérisé par le fait que l'on opère à une température de 60°C à 220°C et de préférence de 100°C à 180°C.

**19 -** Procédé selon l'une des revendications 1 à 18 caractérisé par le fait que le composé aromatique mono- ou polyalkoxylé de formule (I) est l'un des composés suivants :
– le méthoxybenzène
– les diméthoxybenzènes
– les triméthoxybenzènes
– le tétraméthoxy-1,2,4,5 benzène

- l'hexaméthoxybenzène
- les méthoxystyrènes
- le méthoxy-2 chlorobenzène
- les méthoxyfluorobenzènes
- les méthoxydifluorobenzènes
- les méthoxytrifluorobenzènes
- les méthoxytétrafluorobenzènes
- le méthoxy-2 phénol
- le méthoxy-3 phénol
- le méthoxy-4 phénol
- le diméthoxy-2,6 méthyl-4 phénol
- le méthoxy-2 anisole
- le méthoxy-3 anisole
- le méthoxy-4 anisole
- le diméthoxy-2,6 méthyl-4 anisole
- l'acide méthoxy-2 benzoïque
- l'acide méthoxy-3 benzoïque
- l'acide méthoxy-4 benzoïque
- l'ester méthylique de l'acide (diméthoxy-3,5 acétamido-4) benzoïque
- l'ester éthylique de l'acide (diéthoxy-3,5 acétamido-4) benzoïque
- l'ester méthylique de l'acide (triméthoxy-3,4,5) benzoïque
- l'ester éthylique de l'acide (diéthoxy-3,5 méthoxy-4) benzoïque
- l'ester méthylique de l'acide (diméthoxy-3,5 éthoxy-4) benzoïque
- l'ester éthylique de l'acide (triéthoxy-3,4,5) benzoïque
- la méthoxy-4 (trifluorométhyl)-2 acétanilide
- les biphényls mono- ou polyméthoxylés
- les biphényls mono- ou polyéthoxylés
- les bis-phénols méthoxylés ou éthoxylés
- les méthoxyisopropylidène-4,4' bis-phénols
- le méthoxy-2 thiophène
- le méthoxy-3 thiophène
- la méthoxy-2 pyridine
- la méthoxy-3 pyridine
- la méthoxy-4 pyridine

Office européen des brevets

# RAPPORT DE RECHERCHE EUROPEENNE

Numero de la demande

EP 91 40 3155

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5 ) |
|---|---|---|---|
| A | EP-A-0 024 019 (BAYER)<br>* page 3, ligne 9 - page 4, ligne 28; revendication; exemple 1 *<br>--- | 1-19 | C07B41/04<br>C07C43/23<br>C07C43/205<br>C07C43/225 |
| D,A | GB-A-2 089 672 (STERWIN)<br>* revendications; exemples 1-8 *<br>----- | 1-19 | |
| | | | DOMAINES TECHNIQUES RECHERCHES (Int. Cl.5 ) |
| | | | C07C |

Le présent rapport a été établi pour toutes les revendications

| Lieu de la recherche | Date d'achèvement de la recherche | Examinateur |
|---|---|---|
| LA HAYE | 03 MARS 1992 | ZERVAS B. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)